# EUROPÄISCHE PATENTSCHRIFT

(11) **EP 0 548 308 B1**
(45) Veröffentlichungstag und Bekanntmachung des Hinweises auf die Patenterteilung: **28.02.1996**
(21) Anmeldenummer: 92913375.9
(22) Anmeldetag: 01.07.1992
(51) Int. Cl.: C07D 209/14, C07D 209/08

(54) **VERFAHREN ZUR HERSTELLUNG VON INDOL-DERIVATEN**
PROCESS FOR PRODUCING INDOLE DERIVATIVES
PROCEDE DE FABRICATION DE DERIVES DE L'INDOLE

(30) Priorität: 06.07.1991 DE 4122722
(43) Veröffentlichungstag der Anmeldung: 30.06.1993
(73) Patentinhaber: SCHERING AKTIENGESELLSCHAFT, D-13342 Berlin (DE)
(72) Erfinder: HAFFER, Gregor, D-1000 Berlin 45 (DE); BÖRNER, Helmut, D-1000 Berlin 27 (DE); KÜBLER, Wolfgang, D-1000 Berlin 27 (DE); NICKISCH, Klaus, D-1000 Berlin 49 (DE)
(86) Internationale Anmeldenummer: DE9200551
(87) Internationale Veröffentlichungsnummer: WO9301168

(56) Entgegenhaltungen:
- EP-A- 0 054 507
- EP-A- 0 239 667
- DE-A- 4 013 907
- GB-A- 1 079 091
- CHEMICAL ABSTRACTS, vol. 87, no. 3, 18. Juli 1977, Columbus, Ohio, US; abstract no. 22951J, 'indole derivatives' Seite 621 ;
- CHEMICAL ABSTRACTS, vol. 75, no. 9, 30. August 1971, Columbus, Ohio, US; abstract no. 63605V, 'indole derivatives and their intermediates' Seite 437 ;
- CHEMICAL ABSTRACTS, vol. 86, no. 5, 31. Januar 1977, Columbus, Ohio, US; abstract no. 29624T, 'intermediates for indoles' Seite 350 ;

## Beschreibung

Die Erfindung betrifft das Verfahren zur Herstellung von Indol-Derivaten.

Indole sind Zwischenprodukte für die Synthese pharmakologisch anwendbarer Verbindungen wie beispielsweise zur Herstellung von Tryptaminen (C.A. 56, 11701 (1962), Tryptophanen (R.V. Heinzelmann et al. Org. Chemie 25. 1548 (1960) und Carbolinen (EP-A-239667, EP-A-234 173).

Die Herstellung von Indolen kann beispielsweise nach GB-A-1 079 091, Chemical Abstracts, Bd 87, 1977, 22951j, Chemical Abstracts, Bd 75, 1971, 63605v, Chemical Abstracts, Bd 86, 1977, 29624t erfolgen.

Aufgrund ihrer guten Bindungsaffinität an die Benzodiazepin-Rezeptoren zeigen B-Carboline Wirkungen auf das Zentralnervensystem und haben daher in letzter Zeit großes Interesse in der Arzneimittelforschung gefunden.

Es zeigte sich jedoch, daß die Umsetzung des Indols mit Aldehyden und räumlich anspruchsvolleren primären Aminen wie tert. Butyl- bzw. Isopropylamin in Eisessig in schlechten und bei Verwendung der entsprechenden Aldimine in besseren Ausbeuten gelingt, und daß die Ausbeute stark von den Substituenten am Indol beeinflußt wird.

Beispielsweise reagiert das Imin aus Methoxyacetaldehyd und Isopropylamin mit 4- bzw. 5-Benzyloxyindol nur in einer Ausbeute von 60 bzw. 64 % zur Zielverbindung (EP 54 507). Werden jedoch mit Halogen substituierte Phenoxy-indole mit Iminen in Eisessig umgesetzt, so erhält man nicht die gewünschten Gramin-Derivate, sondern isoliert neben der Ausgangsverbindung noch ein dimeres Produkt [G. Neef et al. Heterocycles 20, 1295,1299 [1963].

Es stellte sich daher die Aufgabe, ein Verfahren zu entwickeln, das auf Grund seiner guten Ausbeuten bei guter Handhabung und ohne Aufreinigungsprobleme die großtechnische Herstellung dieser β-Carbolin-Zwischenstufen unabhängig von der Art des Substituenten am Indol ermöglicht.

Überraschenderweise wurde nun gefunden, daß bei der Umsetzung von Indolen mit Iminen in Gegenwart von Phosphorsäure die gewünschten Zwischenprodukte zur Herstellung pharmakologisch wertvoller β-Carboline in guten bis sehr guten Ausbeuten anfallen.

Ein weiterer Vorteil des erfindungsgemäßen Verfahrens besteht darin, daß nur gut abtrennbare und umweltfreundliche Nebenprodukte entstehen.

Beispielsweise enthalten die alkalischen Waschwässer leicht lösliches Dikaliumhydrogenphosphat und aus dem Iminüberschuß Aldehyd, der mit Wasserstoffperoxid zum Kaliumsalz der Carbonsäure oxidiert wird. Anschließend wird das Phosphat als schwer lösliches Kalziumhydrogenphosphat-Dihydrat aus den Waschwässern entfernt.

Die Erfindung betrifft das Verfahren zur Herstellung von Verbindungen der Formel I 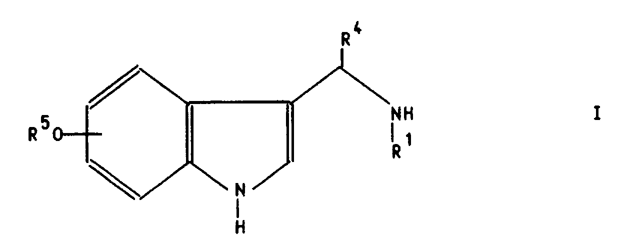 worin
- R¹: C₁₋₄-Alkyl,
- R⁴: Wasserstoff, C₁₋₄-Alkyl oder C₁₋₄-Alkoxy-C₁₋₂ -Alkyl und
- R⁵: C₁₋₄ -Alkyl oder einen gegebenenfalls substituierten Phenyl- oder Phenyl-C₁₋₂-alkyl-Rest bedeutet und -OR⁵ ein- bis mehrfach stehen kann,
dadurch gekennzeichnet, daß man eine Verbindung der Formel II 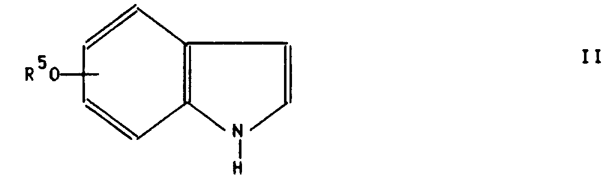 worin R⁵ die oben angegebene Bedeutung hat mit einem Imin der Formel III

R⁴-CH=N-R¹ III,

worin R⁴ und R¹ die obige Bedeutung haben, in Gegenwart von Phosphorsäure umsetzt.

Der Substituent -OR⁵ kann in 4-. 5-, 6- und/oder 7-Stellung ein- oder mehrfach, insbesondere ein- bis zweifach stehen, wobei die einfache Substitution in 4- oder 5-Stellung bevorzugt ist.

Unter C₁₋₄-Alkyl ist jeweils eine gerade oder verzweigte Alkylgruppe zu verstehen wie Methyl, Ethyl, Propyl, Isopropyl, Butyl, sek. Butyl, tert. Butyl und Isobutyl.

Als Phenyl-C₁₋₂ -Alkyl-Rest R⁵ seien beispielsweise Benzyl, Phenethyl und α-Methylbenzyl genannt. Der Phenylrest und der Phenyl-C₁₋₂-Alkyl-Rest können in jeder beliebigen Position ein- oder zweifach substituiert sein, wobei einfache Substitution bevorzugt ist.

Als Substituenten des Phenylrestes seien beispielsweise Halogene, C₁₋₄-Alkyl, C₁₋₄-Alkoxy, Trifluormethyl, Cyano oder Nitro genannt, wobei die Substitution mit Halogenen bevorzugt ist.

Als Substituenten des Phenyl-C₁₋₂-Alkyl-Restes sind insbesondere Halogene, C₁₋₄-Alkyl, C₁₋₄-Alkoxy, Trifluormethyl oder Cyano geeignet, wobei Halogene bevorzugte Substituenten sind.

Halogen beinhaltet jeweils Fluor, Chlor, Brom oder Jod.

Als bevorzugte Substituentenkombination ist gegebenenfalls mit Halogen substituiertes Phenyl und gegebenenfalls mit Halogen substituiertes Benzyl zu betrachten, R⁴ bedeutet vorzugsweise C₁₋₄-Alkoxy-C₁₋₂-Alkyl und R¹ ist insbesondere Isopropyl.

Die erfindungsgemäße Umsetzung wird in Gegenwart molarer Mengen von Phosphorsäure oder mit einem Überschuß an Phosphorsäure bei Temperaturen von - 20 °C bis Raumtemperatur, vorzugsweise bei - 10 °C bis + 10 °C, durchgeführt und ist im allgemeinen nach 1/2 Stunde bis zu 3 Stunden beendet.

Zweckmäßigerweise wird die Phosphorsäure in protischen oder polaren Lösungsmitteln wie Alkoholen oder Ethern, beispielsweise Methanol, Ethanol, Propanol u.a. gelöst und mit equivalenten Mengen oder einem Überschuß an Imin, gegebenenfalls gelöst in einem inerten Lösungsmittel, und mit dem entsprechenden Indol umgesetzt. Als inerte Lösungsmittel kommen Kohlenwasserstoffe, chlorierte Kohlenwasserstoffe und cyclische oder acyclische Ether in Betracht. Die Reaktion kann auch in Suspension erfolgen.

Die Herstellung des neuen Ausgangsverbindungen der Formel IIa 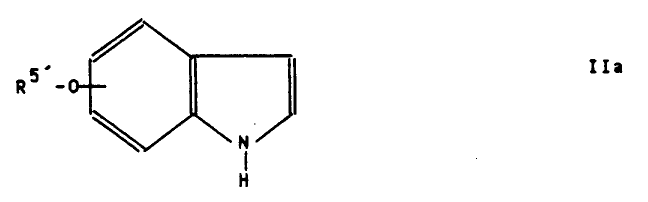 worin R^{5'} gegebenenfalls substituiertes Phenyl bedeutet und -OR^{5'} in 4-oder 5-Stellung steht, erfolgt nach bekannten Methoden, indem man eine Verbindung der Formel IV 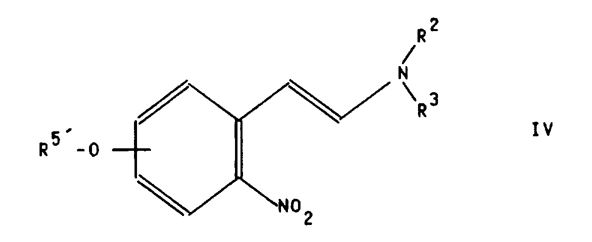 worin R^{5'}die obige Bedeutung hat, und R und R³ gleich oder verschieden sind und jeweils C₁₋₄-Alkyl oder gemeinsam mit dem Stickstoffatom einen gegebenenfalls ein weiteres Stickstoff-, Sauerstoff-oder Schwefelatom enthaltenden gesättigten Fünf- oder Sechsring bedeuten, katalytisch reduziert und cyclisiert.

Als gesättigte Fünf- oder Sechsringe seien beispielsweise genannt: Imidazolidin, Pyrrolidin, Piperidin, Morpholin, Thiomorpholin, Piperazin.

Als Katalysatoren für die erfindungsgemäße Reduktion eignen sich die üblicherweise verwendeten Hydrierungskatalysatoren wie beispielsweise Raney-Nickel oder Edelmetallkatalysatoren wie Platinoxid oder Palladium/ Kohle. Die Cyclisierung erfolgt bei Raumtemperatur oder unter Erwärmen bis 50 °C bei Wasserstoffnormaldruck oder erhöhtem Wasserstoffdruck in einer wässrigen Suspension oder in inerten Lösungsmitteln wie Alkoholen, Estern oder Ketonen oder Gemischen derselben. Im Verlaufe der erfindungsgemäßen Umsetzung wird die NRR³-Gruppe abgespalten (W. Leimgruber und A.D. Batcho, Org. Synth. 63. 214 (1985)).

Die Verbindungen der Formel I werden zu den in EP-A-54 507, EP-A-239 667, EP-A-234 173 und EP-A-130 140 beschriebenen Verbindungen nach den dort beschriebenen Methoden weiterverarbeitet.

Die Herstellung der Ausgangsverbindungen ist bekannt oder erfolgt nach bekannten oder hier beschriebenen Methoden.

Die nachfolgenden Beispiele sollen das erfindungsgemäße Verfahren erläutern.

### Herstellung der Ausgangsverbindungen:

### a) 4-(4-Chlorphenoxy)-indol

6-Amino-2-nitrotuluol wird in wässriger 48 proz. Bromwasserstoffsäure mit Natriumnitrit diazotiert und nach Sandmeyer mit Kupfer(I)-bromid in das gewünschte 6-Brom-2-nitro-toluol überführt. Unter modifizierten Ullmann-Bedingungen (Kupfer(I)-chlorid, Zusatz eines festflüssigen Phasentransferkatalysators wie TDA-1 [Tris-(3,6-dioxaheptyl)-amin]) wird 6-Brom-2-nitro-toluol mit dem Kaliumsalz des 4-Chlorphenols in einem hochsiedenden Lösungsmittel zum Diarylether 6-(4-Chlorphenoxy)-2-nitrotoluol umgesetzt (G. Soula, J. Org. Chem. 50, 3717 (1985)).

In Analogie zur Methode nach Leimgruber-Batcho wird aus 6-(4-Chlorphenoxy)-2-nitrotoluol ohne Isolierung das 6-(4-Chlorphenoxy)-2-nitro-β-pyrrolidinostyrol reduktiv mit Raney-Nickel/Wasserstoff zum 4-(4-Chlorphenoxy)-indol cyclisiert.

### b) N-Isopropyl-N-(2-methoxyethyliden)-amin

144 ml Isopropylamin werden in 225 ml Toluol unter Stickstoffatmosphäre gelöst. Unter Eiswasserkühlung und Rühren tropft man innerhalb von 40 Minuten 112,5 ml Methoxyacetaldehyd hinzu und läßt 30 Minuten unter Kühlung nachrühren. Nach Abstellen des Rührers wird das Reaktionsvolumen 30 Minuten mit Eiswasser gekühlt, danach die untere Phase abpipettiert. Nach portionsweiser Zugabe von 75 g Kaliumkarbonat läßt man die Toluollösung weitere 3 Stunden unter Stickstoff nachrühren, filtriert das Kaliumkarbonat ab und wäscht es dreimal mit je 130 ml Toluol nach. Die Lösung, auf 800 ml Gesamtvolumen mit Toluol aufgefüllt, wird im Tiefkühlschrank bis zur Verwendung aufbewahrt.

### c) N-Isopropyl-N-methylenamin

Aus 144,0 ml Isopropylamin und 125,2 ml einer 37%igen wässrigen Formaldehydlösung erhält man wie unter b) beschrieben 750 ml einer Lösung von N-Isopropyl-n-methylenamin in Toluol.

### d) N-Ethvliden-N-isopropylamin

48,0 ml Isopropylamin und 31,6 ml Acetaldehyd werden analog b) umgesetzt. Man erhält das Imin des Acetaldehyds in 225 ml Toluol gelöst.

### e) N-Isopropyl-N-propylidenamin

72,0 ml Isopropylamin und 60,3 ml Propionaldehyd werden analog b) zu 420 ml einer Lösung des Imins in Toluol umgesetzt.

### Beispiel 1

17,86 g 100%ige Phosphorsäure werden in 32,2 ml Ethanol bei Raumtemperatur gelöst und auf - 5 °C gekühlt. 9,75 g 4-(4-Chlorphenoxy)-indol, in 32,4 ml Iminlösung gelöst, werden innerhalb von 30 Minuten bei einer Innentemperatur unterhalb + 10 °C zur gekühlten Lösung der Phosphorsäure getropft. Man läßt 1 Stunde bei + 10 °C nachrühren. Nach Zugabe von 35,7 ml Toluol und 71,5 ml Wasser werden die Phasen 15 Minuten kräftig durchmischt, die separierte Toluolphase einmal mit 18,0 ml Wasser gewaschen. Die vereinigten Wasserphasen werdem im Vakuum bei + 40°C Badtemperatur auf 25 ml Volumen eingeengt, die Restlösung mit 25 ml Wasser verdünnt. Das gekühlte Reaktionsgemisch wird mit 50%iger Kalilauge auf pH 9.5 eingestellt, der ausgefallene Niederschlag abgesaugt, mit Wasser gewaschen und im Vakuum bei Raumtemperatur über KOH bis zur Gewichtskonstanz getrocknet. Man erhält 6,0 g Ausbeute (42% der Theorie) N-(1-[4-(4-Chlorphenoxy)-indol-3-yl]-2-methoxyethyl)-N-isopropylamin vom Schmelzpunkt 149,4 - 150 °C.

### Beispiel 2

Analog Beispiel 1 erhält man aus 34,47 g 4-Benzyloxy-indol 43,2 g (Ausbeute 82,7% der Theorie) N-[1-(4-Benzyloxy-indolyl-3-yl)-2-methoxy-ethyl]-N-isopropylamin vom Schmelzpunkt 138,4 - 139,3°C.

### Beispiel 3

Durch Zugabe von 22,3 g 5-Benzyloxy-indol und Imin als Suspension wie im Beispiel 1 angegeben zur Phosphorsäurelösung in Ethanol werden 24,35 g (72% der Theorie) des 5-Benzyloxyindol-pseudogramins vom Schmelzpunkt 108,7 - 110,3 °C isoliert.

### Beispiel 4

Durch Umsetzen von 6,09 g 4-(4-Chlorphenoxy)-indol mit 19,05 ml Iminlösung c) werden wie in Beispiel 1 beschrieben 3,70 g (47,0 % der Theorie)

N- 1-(4-[-Chlorphenoxy]-indol-3-yl)-methyl -N-isopropylamin vom Schmelzpunkt 88,2 - 90.0 °C erhalten.

### Beispiel 5

Analog Beispiel 1 erhält man aus 50,0 g 4-Benzyloxyindol und 144,0 ml Iminlösung d) 39,2 g (65,8 % der Theorie) N-[1-(4-Benzyloxyindol-3-yl)-ethyl]-N-isoppropylamin vom Schmelzpunkt 143,0 °C.

### Beispiel 6

Durch Zugabe von 39,7 g 5,6-Dimethoxyindol und 190,5 ml Iminlösung e) als Suspension zur Phosphorsäure gelöst in Ethanol werden in Analogie zum Beispiel 1 37,15 g N-Isopropyl-N-[1-(5,6-dimethoxyindol-3-yl)-propyl]-amin (60,0 % der Theorie) vom Schmelzpunkt 124.0 - 126,9 °C erhalten.

## Patentansprüche

1. Verfahren zur Herstellung von Verbindungen der Formel I 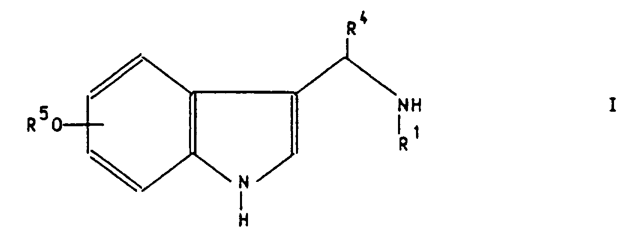 worin
R¹ C₁₋₄-Alkyl,
R⁴ Wasserstoff, C₁₋₄-Alkyl oder C₁₋₄-Alkoxy-C₁₋₂ -Alkyl und
R⁵ C₁₋₄-Alkyl oder einen gegebenenfalls mit Halogenen, C₁₋₄-Alkyl, C₁₋₄-Alkoxy, Trifluormethyl, Cyano oder Nitro substituierten Phenyl-oder Phenyl-C₁₋₂-alkyl-Rest bedeutet und -OR⁵ ein- bis mehrfach stehen kann,
dadurch gekennzeichnet, daß man eine Verbindung der Formel II 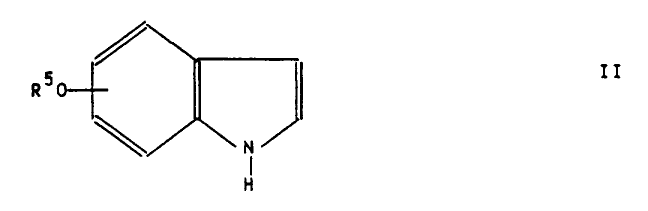 worin R⁵ die oben angegebene Bedeutung hat mit einem Imin der Formel III
R⁴-CH=N-R¹ III,
worin R⁴ und R¹ die obige Bedeutung haben, in Gegenwart von Phosphorsäure umsetzt.

## Claims

1. A process for the preparation of compounds of formula I 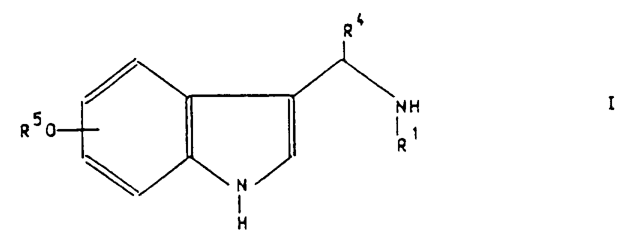 wherein
R¹ is C₁₋₄alkyl,
R⁴ is hydrogen, C₁₋₄alkyl or C₁₋₄alkoxy C₁₋₂alkyl, and
R⁵ is C₁₋₄alkyl; or a phenyl or phenyl-C₁₋₂alkyl radical each optionally substituted by halogen, C₁₋₄alkyl, C₁₋₄alkoxy, trifluoromethyl, cyano or by nitro, and -OR⁵ can be present one or more times,
characterised in that a compound of formula II 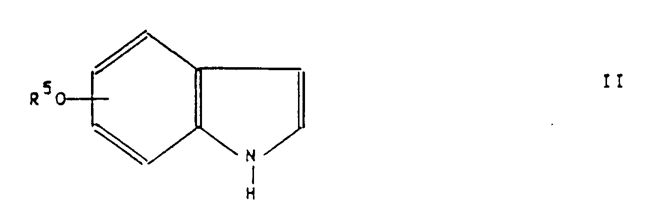 wherein R⁵ has the meaning given above is reacted in the presence of phosphoric acid with an imine of formula III
R⁴-CH=N-R¹ III
wherein R⁴ and R¹ have the meaning given above.

## Revendications

1. Procédé de préparation de composés de formule I : 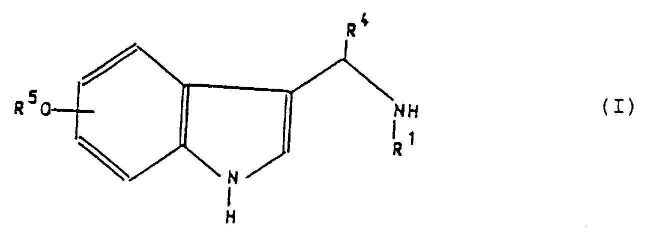 dans laquelle
R¹ désigne un alkyle en C₁-C₄,
R⁴ l'hydrogène, un alkyle en C₁-C₄ ou un (alcoxy en C₁-C₄)-alkyle en C₁-C₂ et
R⁵ un alkyle en C₁-C₄ ou bien un reste phényle ou phényl -alkyle en C₁-C₂ éventuellement substitués par des halogènes, alkyles en C₁-C₄, alcoxy en C₁-C₄, trifluorométhyle, cyano ou nitro, avec la possibilité qu'il y ait un seul ou plusieurs groupes -OR⁵,
procédé caractérisé en ce que l'on fait réagir un composé de formule II : 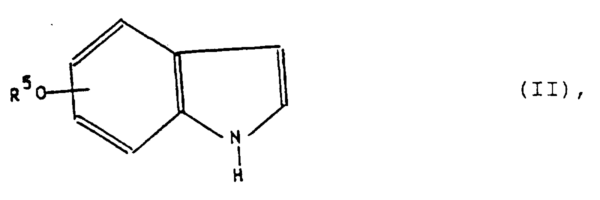 dans laquelle R⁵ a la signification mentionnée ci-dessus avec une imine de formule III :
R⁴-CH=N-R¹ (III),
dans laquelle R⁴ et R¹ ont les signification ci-dessus, en présence d'acide phosphorique.
